(19) Europäisches Patentamt
European Patent Office
Office européen des brevets

(11) **EP 2 526 867 B1**

(12) **EUROPEAN PATENT SPECIFICATION**

(45) Date of publication and mention
of the grant of the patent:
**18.04.2018 Bulletin 2018/16**

(51) Int Cl.:
**A61B 6/00** (2006.01)

(21) Application number: **11305640.2**

(22) Date of filing: **26.05.2011**

(54) **X-ray imaging apparatus having vibration stabilising means, and method for operating such an X-ray imaging apparatus**

Röntgenbildgebungsvorrichtung mit vibrationsstabilisierenden Mitteln und Verfahren zum Betrieb solch einer Röntgenbildgebungsvorrichtung

Appareil d'imagerie par rayons X doté d'un moyen de stabilisation des vibrations et procédé pour faire fonctionner un tel appareil

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**28.11.2012 Bulletin 2012/48**

(73) Proprietor: **General Electric Company**
**Schenectady, NY 12345 (US)**

(72) Inventors:
• **Martinez Ferreira, Carlos**
  **78533 Buc (FR)**

• **Al Assad, Omar**
  **78533 Buc (FR)**

(74) Representative: **Dossmann, Gérard**
**Casalonga & Partners**
**Bayerstrasse 73**
**80335 München (DE)**

(56) References cited:
**JP-A- 2003 245 269    JP-A- 2005 027 914**

EP 2 526 867 B1

**Description**

The present invention relates in general to medical imaging systems and, notably, to medical imaging systems minimizing the vibrations generated by such imaging systems when operated.

In one particular application, the invention relates to the active control of vibrations generated within imaging systems used for angiographic examinations for diagnostic or interventional purposes.

Imaging systems usually comprise an imaging assembly comprising an X-ray source, namely an X-ray tube, and an X-ray detector placed opposite to the X-ray tube in a direction of emission of the X-rays. The tube and the detector are usually placed on two mutually opposite ends of a so-called "C-arm" shaped in the form of an arch.

During a radiographic examination, it is necessary to produce radiographs of a region of interest in the body of a patient irradiated by X-rays. For this purpose, after the patient has been laid out on an examination table, the X-ray tube and the detector are brought to face the region to be radiographed and to be centered around the region of interest.

When the X-ray chain, comprising the X-ray tube and the X-ray detector is moved to reach the region of interest, or when the X-ray chain is stopped, vibrations may appear in particular within the imaging assembly. However, the image quality and the perception of the image viewed by the operator are highly damaged when vibrations occur.

It is therefore highly desirable to eliminate the vibrations generated when the X-ray chain is moved to the region of interest or stopped.

In the prior art, vibration absorbers are known, permitting to control vibrations in an imaging system.

Reference can be made to document US 5,548,653.

The vibration control system disclosed in this document is in particular intended to minimize the noise and/or vibrations generated by a magnetic resonance imaging (MRI) system, and is operated to create a secondary noise and/or vibration field which cancels the primary noise field generated when the MRI system is operated.

Reference can also be made to document US 5,348,124, in which low frequency vibrations are prevented from propagating along an elongate member using magnetostrictive devices.

Other references, namely US 6,959,484, US 2002/0008362, US 6,002,778, US 2002/0128072 and US 2007/0164189 disclose vibration isolation devices in various technical fields, for example within aircrafts or automotive vehicles.

The vibration absorbers according to the state of the art, and in particular as disclosed in document US 5,548,653, are intended to absorb vibrations in the MRI system as a whole. It has thus been noted that such vibration absorbers are not suitable to absorb efficiently the vibrations generated within an imaging assembly when moved to reach a region of interest or subsequently stopped.

Document JP2005027914 discloses a C-arm with a vibration generator placed on the C-arm structure and close to the detector, used to compensate vibrations detected by a vibration sensor.

Document JP2003245269 discloses an X-ray imaging device with vibration compensation means placed on the supporting platforms for the X-ray source and the detector.

In the light of the foregoing, the object of the invention is to propose an X-ray imaging apparatus and a method for operating such an X-ray imaging apparatus which remediates this drawback and permittes to suppress the visible vibrations within the image.

The subject of the invention is therefore an X-ray imaging apparatus, comprising a rotary arm and an imaging assembly mounted on the rotary arm and comprising an X-ray source and an X-ray detector coupled to the rotary arm so that X-rays emitted by the X-ray source are incidental to the detector.

According to a general feature of the invention, the X-ray imaging apparatus further comprises first and second vibration measurement means coupled to the X-ray source and to the X-ray detector, respectively, and first and second actuators suitable for moving said X-ray source and X-ray detector to suppress at least one component of the vibrations measured by said vibration measurement means.

By using vibration measurement means and an actuator associated with the X-ray source, on the one hand, and with the X-ray detector, on the other hand, it is possible to minimize vibrations in the X-ray source and in the X-ray detector, separately.

In addition, when suppressing the vibrations, the detector and the tube alignment during all the imaging process is guaranteed.

According to another feature, the first actuator comprises a first piezoelectric actuator coupled between the rotary arm and the X-ray source, and the second actuator comprises a second piezoelectric actuator coupled between the rotary arm and the X-ray detector.

In one embodiment, the first and second measurement means comprise first and second accelerometers coupled to the X-ray source and to the X-ray detector, respectively.

For example, the first and second accelerometers are mounted on the X-ray source and on the X-ray detector, respectively.

According to another feature, the X-ray imaging apparatus comprises a vibration controller receiving vibration level measures delivered by the first and second measurement means, and suitable for elaborating first and second control signals for the first and second actuators from the vibration level measures.

According to yet another feature, the vibration controller is suitable for generating said first and second control signals to minimize the following function:

$$\Delta i = \left(Act_d + F_2 + F_1\right) + \left(Act_t + F_3 + F_1\right)\frac{P_d - P_t}{P_p - P_t} \qquad (1)$$

wherein:

- $\Delta i$ corresponds to the image variation due to the vibrations ;
- $Act_d$ represents the difference between the position of the X-ray detector and the position of the second actuator ;
- $F_1$ corresponds to the position of a knuckle of the rotary arm;
- $F_2$ corresponds to the difference of position between the second actuator and the knuckle ;
- $Act_t$ represents the difference between the position of the X-ray source and the position of the first actuator ;
- $F_3$ represents the difference of position between the first actuator and the knuckle ; and
- $P_d$, $P_t$ and $P_p$ correspond to the position of the X-ray detector, of the X-ray source and of the patient body to be imaged, respectively.

A further subject of the invention, according to a second aspect, is a method for operating an X-ray imaging apparatus as defined above, comprising the steps of :

- measuring vibrations in the X-ray source and in the X-ray detector ; and
- actuating the first and second actuators in order to move the X-ray source and the X-ray detector to suppress at least one component of the vibrations.

In one embodiment, this method further comprises elaborating first and second control signals for the first and second actuators from a vibration measure level.

Other objects, features and advantages of the invention will appear on reading the following description, given only as a non-limiting example, and made with reference to the appended drawings, in which:

- figure 1 is a schematic view of an X-ray imaging apparatus according to the invention;
- figure 2 is a schematic view of the X-ray imaging apparatus of figure 1, illustrating transfer functions used to elaborate the control signals for the actuators;
- figure 3 is a schematic view of the vibration controller; and
- figure 4 illustrates a vibration image control diagram corresponding to the vibration suppression method carried out by the vibration controller.

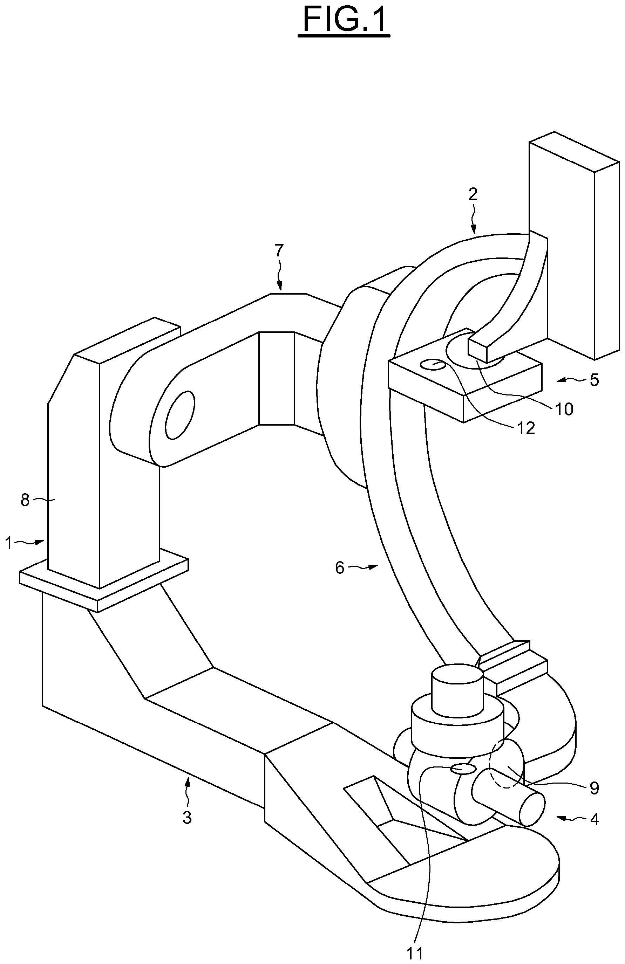

Reference is first made to figure 1, which illustrates a medical imaging apparatus according to one embodiment of the invention, of the vascular type.

In this envisaged but non-limiting application, this imaging system 1 is intended to be used for angiographic examination.

As it can be seen, this apparatus 1 essentially comprises an imaging assembly 2 mounted on a base assembly 3.

The imaging assembly 2 essentially comprises an X-ray tube 4, capable of emitting a beam of X-rays in an emission direction, and an X-ray detector 5 placed at two mutually opposed ends of a C-arm 6, in this instance in the form of an arch, so that the X-rays emitted by the tube 4 are incident on the detector 5.

The arm 6 is mounted so as to slide on a second arm 7 mounted rotatingly on a fixed support 8, itself mounted on the base assembly 3.

Therefore, the support 8, the rotating arm 7 and the C-arm 6 are all three articulated relative to one another so that the X-ray imaging apparatus can move in three dimensions so as to produce images of an organ to be examined from various angles.

During a radiography, the tube 4 and the detector 5 are brought to face a region of interest (ROI) in the body of a patient laid out on an examination table (not shown) so that, when the region of interest is interposed between the X-ray tube 4 and the detector 5, it is irradiated by the X-rays, and the detector 5 produces data representative of characteristics of the interposed region of interest.

In order to suppress the vibrations generated by the imaging assembly movements or residual vibrations generated when the imaging assembly has reached its final position and is centered around the region of interest, the X-ray imaging apparatus is further provided with vibration measurement means and actuators operable to locally move the tube 4 and the detector 5, to locally control their position.

As seen, the imaging assembly thus comprises two actuators 9, 10, the one for the tube 4 and the other for the detector 5, and two vibration measurement means 11 and 12, the one for the tube 4 and the other for the detector 5.

The vibration measurement means are preferably 3-axes measurement means and are directly attached to the tube 4 and to the detector 5, respectively.

The object of the vibration measurement means is to measure vibration levels in order to elaborate first and second control signals for the actuators 9 and 10.

It is understood that the vibration measurement means can include different arrangements and may for example comprise two accelerometers mounted on the tube and on the detector, respectively.

The actuators 9 and 10 are coupled between the C-arm 6 and the tube and the detector, respectively. They are mounted to the mutual opposite ends of the C-arm, in place of the rigid coupling used within the X-ray imaging apparatus according to the state of the art.

The object of the two actuators is to locally move the tube and the detector to suppress the vibrations that may be visible within the image generated by the image assembly.

As can be conceived, the two actuators 9 and 10 may also be of different arrangements. For example, in a particular embodiment, each actuator 9 and 10 has piezoelectric properties, such that, when excited by the first and second control signals, it exerts a displacement force on the actuator to which it is attached.

In other words, the actuators each consist in a piezoelectric actuator mounted to one end of the C-arm 6, and to which is attached the tube 4 or the detector 5, such that, when excited, the thickness of the actuators 9 and 10 is modified in response to the first and second control signals and the position of the tube and of the detector can be modified in a way to suppress the visible vibrations.

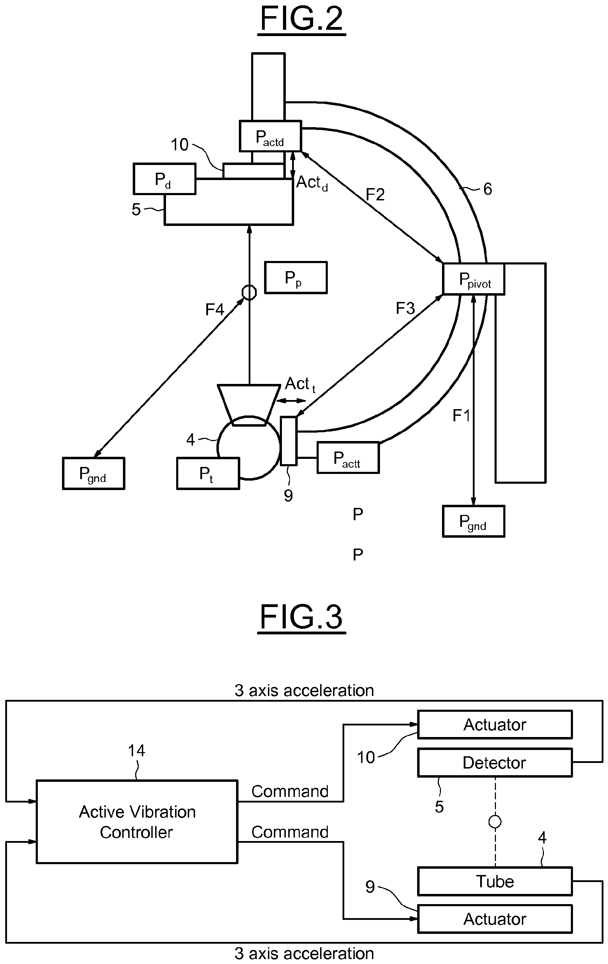

Referring now to figure 2, the X-ray imaging apparatus may be provided with a set of sensors to measure the position of the significant elements of the apparatus, namely a sensor $P_d$ for measuring the position of the detector; a sensor $P_{actd}$ for measuring the position of the second actuator; a sensor $P_{pivot}$ for measuring the position of the knuckle of the C-arm 6; a sensor $P_p$ for measuring the position of the region of interest of the patient; a sensor $P_t$ for measuring the position of the tube; and a sensor $P_{actt}$ for measuring the position of the first actuator.

In addition, a set of transfer functions may be calculated by the vibration controller, namely:

- a transfer function $F_1$ between the ground position $P_{gnd}$ and the knuckle position $P_{pivot}$;
- a transfer function $F_2$ between the knuckle position $P_{pivot}$ and the second actuator detector position $P_{actd}$;
- a transfer function $F_3$ between the knuckle position $P_{pivot}$ and the first actuator tube position $P_{actt}$;
- a transfer function $F_4$ between the ground position $P_{gnd}$ and the object position $P_p$;
- a transfer function $A_{ctt}$ between the tube position and the first actuator position $P_{actt}$; and
- a transfer function $A_{ctd}$ between the detector position $P_d$ and the second actuator position $P_{actd}$.

In other words, the transfer functions reads as follows:

$$P_{pivot} - P_{gnd} = F_1 \qquad (1)$$

$$P_{Actd} - P_{pivot} = F_2 \qquad (2)$$

$$P_{Actt} - P_{pivot} = F_3 \qquad (3)$$

$$P_d - P_{Actd} = Act_d \qquad (4)$$

$$P_t - P_{Actt} = Act_t \qquad (5)$$

Taking as reference the ground, ($P_{gnd}$ = 0), the following transfer functions are obtained:

$$P_{Actd} - F_1 = F_2 \qquad (6)$$

$$P_{Actt} - F_1 = F_3 \qquad (7)$$

and :

$$P_d = Act_d + F_2 + F_1 \qquad (8)$$

$$P_t = Act_t + F_3 + F_1 \qquad (9)$$

In view of the foregoing, the variation $\Delta i$ in the image due to vibrations may be defined by the following equation:

$$\Delta i = \Delta P_d + \Delta P_t \left( \frac{P_d - P_t}{P_p - P_t} \right) \qquad (10)$$

such that:

$$\Delta i = \Delta \left[ Act_d + F_2 + F_1 \right] + \Delta \left[ Act_t + F_3 + F_1 \right] \left( \frac{P_d - P_t}{P_p - P_t} \right) \qquad (11)$$

It should be appreciated that, as illustrated by figure 3, the vibration levels on the tube and detector measured by the sensors $P_{Actd}$ and $P_{Actt}$ are used as a feedback data to elaborate the first and second control signals *"command"* to move locally the tube and the detector and suppress the visible vibrations.

It should also be noted that the vibration controller 14 may be, for example, incorporated within a central processing unit. Such a central processing unit may be furnished with storage means, of data storage memory type, for example of the ROM, RAM, etc... type, incorporating one or more control algorithm capable of moving the imaging assembly relating to the base assembly, in particular the rotation of the C-arm, either automatically or under the control of a control console (not shown), in response to instructions entered manually by an operator.

In view of the foregoing, the vibration controller calculates the transfer function $Act_t$ and $Act_d$ in order to minimize the image vibration cost function:

$$\min \left\{ \left( Act_d + F_2 + F_1 \right) + \left( Act_t + F_3 + F_1 \right) \frac{P_d - P_t}{P_p - P_t} \right\} \qquad (12)$$

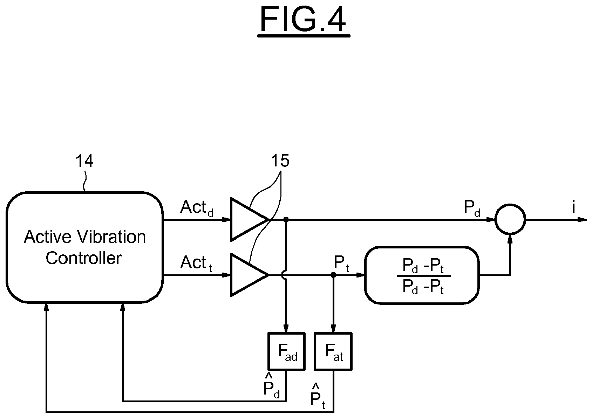

Referring now to figure 4, illustrating a control diagram carried out by the vibration controller to calculate equations (11) and (12) elaborates transfer functions $Act_d$ and $Act_t$ in order to minimize the vibration within the image and therefore the variation in the image due to vibrations.

The thus calculated transfer functions are amplified by amplifiers 15 and 16 to generate the first and second control signals *Fad* and *Fat* used to drive the actuators. As seen, an estimate $\hat{P}_d$ and $\hat{P}_t$ of the position of the tube and of the detector is used as a feedback loop by the vibration controller 14.

It will be understood that, by virtue of the imaging apparatus comprising a rotary arm and an imaging assembly mounted on the rotary arm and comprising an X-ray source and an X-ray detector coupled to the rotary arm so that the X-rays emitted by the X-ray source are incidental to the detector, and comprising first and second accelerometers coupled to the X-ray source and to the X-ray detector, respectively, and first and second piezoelectric actuators suitable for moving the X-ray source and the X-ray detector, it is possible to suppress at least one component of the vibrations measured by the first second accelerometers.

**Claims**

1.   X-ray imaging apparatus, comprising a rotary arm (6) and an imaging assembly (2) mounted on the rotary arm and comprising an X-ray source (4) and an X-ray detector (5) coupled to the rotary arm so that X-rays emitted by the X-ray source are incidental to the detector, the apparatus further comprising first and second vibration measurement means (11, 12) coupled to the X-ray source and to the X-ray detector, respectively, and first and second actuators (9, 10) suitable for moving said X-ray source and said X-ray detector to suppress at least one component of the vibrations measured by said vibration measurement means,

wherein the first actuator (9) comprises a first piezoelectric actuator coupled between the rotary arm and the X-ray source and the second actuator (10) comprises a second piezoelectric actuator coupled between the rotary arm and the X-ray detector, and

wherein a thickness of the actuators (9, 10) is modified to suppress the at least one component of the vibrations measured by said measurement means.

2. X-ray imaging apparatus according to claim 1, wherein the first and second measurement means comprise first and second accelerometers (11, 12) coupled to the X-ray source and to the X-ray detector, respectively.

3. X-ray imaging apparatus according to claim 2, wherein said first and second accelerometers are mounted on the X-ray source and on the X-ray detector, respectively.

4. X-ray imaging apparatus according to any of claims 1 to 3, further comprising a vibration controller (14) receiving vibration level measures delivered by the first and second measurement means and suitable for elaborating first and second control signals for the first and second actuators from the vibration level measures.

5. X-ray imaging apparatus according to claim 4, wherein the vibration controller is suitable for generating said first and second control signals to minimize the following function:

$$\Delta i = \left(Act_d + F_2 + F_1\right) + \left(Act_t + F_3 + F_1\right)\frac{P_d - P_t}{P_p - P_t} \qquad (1)$$

wherein:

- $\Delta i$ corresponds to the image variation due to the vibrations;
- $Act_d$ represents the difference between the position of the X-ray detector and the position of the second actuator;
- $F_1$ corresponds to the position of a knuckle of the rotary arm;
- $F_2$ corresponds to the difference of position between the second actuator and the knuckle;
- $Act_t$ represents the difference between the position of the X-ray source and the position of the first actuator;
- $F_3$ represents the difference of position between the first actuator and the knuckle; and
- $P_d$, $P_t$ and $P_p$ correspond to the position of the X-ray detector, of the X-ray source and of the patient body to be imaged, respectively.

6. Method for operating an X-ray imaging apparatus according to any of claims 1 to 5, **characterized in that** it comprises the steps of:

- measuring vibrations in the X-ray source and in the X-ray detector; and
- actuating the first and second actuators in order to move the X-ray source and the X-ray detector to suppress at least one component of the vibrations.

7. Method according to claim 6, further comprising elaborating first and second control signals for the first and second actuators.

**Patentansprüche**

1. Röntgenstrahlbildgebungsvorrichtung, die einen Dreharm (6) und eine Bildgebungsanordnung (2), die auf dem Dreharm angebracht ist und eine Röntgenstrahlquelle (4) umfasst, und einen Röntgenstrahldetektor (5), der an den Dreharm gekoppelt ist, so dass durch die Röntgenstrahlquelle ausgesendete Röntgenstrahlen auf den Detektor einfallen, umfasst, wobei die Vorrichtung ferner erste und zwei Schwingungsmessmittel (11, 12), die jeweils an die Röntgenstrahlquelle und an den Röntgenstrahldetektor gekoppelt sind, und ein erstes und ein zweites Betätigungselement (9, 10), die geeignet sind, um die Röntgenstrahlquelle und den Röntgenstrahldetektor zu bewegen, um mindestens eine Komponente der durch die Schwingungsmessmittel gemessenen Schwingung zu unterdrücken, umfasst,

wobei das erste Betätigungselement (9) ein erstes piezoelektrisches Betätigungselement, das zwischen den Dreharm und die Röntgenstrahlquelle gekoppelt ist, umfasst und das zweite Betätigungselement (10) ein zweites piezoelektrisches Betätigungselement, das zwischen den Dreharm und den Röntgenstrahldetektor gekoppelt ist, umfasst und

wobei eine Dicke der Betätigungselemente (9, 10) geändert wird, um die mindestens eine Komponente der durch die Messmittel gemessenen Schwingungen zu unterdrücken.

2. Röntgenstrahlbildgebungsvorrichtung nach Anspruch 1, wobei die ersten und die zweiten Messmittel einen ersten und einen zweiten Beschleunigungsmesser (11, 12) umfassen, die jeweils an die Röntgenstrahlquelle und den Röntgenstrahldetektor gekoppelt sind.

3. Röntgenstrahlbildgebungsvorrichtung nach Anspruch 2, wobei der erste und der zweite Beschleunigungsmesser jeweils auf der Röntgenstrahlquelle und dem Röntgenstrahldetektor angebracht sind.

4. Röntgenstrahlbildgebungsvorrichtung nach einem der Ansprüche 1 bis 3, die ferner eine Schwingungssteuereinheit (14) umfasst, die Schwingungspegelmessungen empfängt, die durch die ersten und die zweiten Messmittel geliefert werden, und die geeignet ist, ein erstes und ein zweites Steuersignal für das erste und das zweite Betätigungselement aus den Schwingungspegelmessungen auszuarbeiten.

5. Röntgenstrahlbildgebungsvorrichtung nach Anspruch 4, wobei die Schwingungssteuereinheit geeignet ist, das erste und das zweite Steuersignal zu erzeugen, um die folgende Funktion zu minimieren:

$$\Delta i = (Act_d + F_2 + F_1) + (Act_t + F_3 + F_1)\frac{P_d - P_t}{P_p - P_t}$$

wobei:

- $\Delta i$ der Bildvariation aufgrund der Schwingungen entspricht;
- $Act_d$ den Unterschied zwischen der Position des Röntgenstrahldetektors und der Position des zweiten Betätigungselements repräsentiert;
- $F_1$ der Position eines Gelenks des Dreharms entspricht;
- $F_2$ dem Unterschied der Position zwischen dem zweiten Betätigungselement und dem Gelenk entspricht;
- $Act_i$ den Unterschied zwischen der Position der Röntgenstrahlquelle und der Position des ersten Betätigungselements repräsentiert;
- $F_3$ den Unterschied der Position zwischen dem ersten Betätigungselement und dem Gelenk repräsentiert; und
- $P_d$, $P_t$ und $P_p$ jeweils der Position des Röntgenstrahldetektors der Röntgenstrahlquelle und dem abzubildenden Patientenkörper entspricht.

6. Verfahren zum Betreiben einer Röntgenstrahlbildgebungsvorrichtung nach einem der Ansprüche 1 bis 5, **dadurch gekennzeichnet, dass** es die folgenden Schritte umfasst:

- Messen von Schwingungen in der Röntgenstrahlquelle und in dem Röntgenstrahldetektor; und
- Betätigen des ersten und des zweiten Betätigungselements, um die Röntgenstrahlquelle und den Röntgenstrahldetektor zu bewegen, um mindestens eine Komponente der Schwingungen zu unterdrücken.

7. Verfahren nach Anspruch 6, das ferner umfasst, ein erstes und ein zweites Steuersignal für das erste und das zweite Betätigungselement auszuarbeiten.

**Revendications**

1. Appareil d'imagerie par rayons X, comprenant un bras rotatif (6) et un ensemble d'imagerie (2) monté sur le bras rotatif et comprenant une source de rayons X (4) et un détecteur de rayons X (5) couplé au bras rotatif de telle manière que les rayons X émis par la source de rayons X arrivent sur le détecteur, l'appareil comprenant en outre un premier et un deuxième moyen de mesure de vibrations (11, 12) couplés respectivement à la source de rayons X et au détecteur de rayons X, et des premier et deuxième actionneurs (9, 10) appropriés pour déplacer ladite source de rayons X et ledit détecteur de rayons X afin de supprimer au moins une composante des vibrations mesurées par lesdits moyens de mesure de vibrations,

dans lequel le premier actionneur (9) comprend un premier actionneur piézoélectrique monté entre le bras rotatif et la source de rayons X et le deuxième actionneur (10) comprend un deuxième actionneur piézoélectrique monté entre le bras rotatif et le détecteur de rayons X, et

dans lequel on modifie une épaisseur des actionneurs (9, 10) pour supprimer ladite au moins une composante des vibrations mesurées par lesdits moyens de mesure.

2. Appareil d'imagerie par rayons X selon la revendication 1, dans lequel les premier et deuxième moyens de mesure comprennent des premier et deuxième accéléromètres (11, 12) couplés respectivement à la source de rayons X et au détecteur de rayons X.

3. Appareil d'imagerie par rayons X selon la revendication 2, dans lequel lesdits premier et deuxième accéléromètres sont montés respectivement sur la source de rayons X et sur le détecteur de rayons X.

4. Appareil d'imagerie par rayons X selon l'une quelconque des revendications 1 à 3, comprenant en outre un contrôleur de vibrations (14) recevant des mesures de niveaux de vibrations délivrées par les premier et deuxième moyens de mesure et approprié pour élaborer des premier et deuxième signaux de commande pour les premier et deuxième actionneurs à partir des mesures de niveaux de vibrations.

5. Appareil d'imagerie par rayons X selon la revendication 4, dans lequel le contrôleur de vibrations est approprié pour générer lesdits premier et deuxième signaux de commande afin de minimiser la fonction suivante :

$$\Delta i = \left( Act_d + F_2 + F_1 \right) + \left( Act_t + F_3 + F_1 \right) \frac{P_d - P_t}{P_p - P_t}$$

dans laquelle :

- $\Delta i$ correspond à la variation d'image due aux vibrations ;
- $Act_d$ représente la différence entre la position du détecteur de rayons X et la position du deuxième actionneur ;
- $F_1$ correspond à la position d'une articulation du bras rotatif ;
- $F_2$ correspond à la différence de position entre le deuxième actionneur et l'articulation ;
- $Act_t$ représente la différence entre la position de la source de rayons X et la position du premier actionneur ;
- $F_3$ représente la différence de position entre le premier actionneur et l'articulation ; et
- $P_d$, $P_t$ et $P_p$ correspondent respectivement à la position du détecteur de rayons X, de la source de rayons X et du corps du patient à examiner.

6. Procédé de mise en oeuvre d'un appareil d'imagerie par rayons X selon l'une quelconque des revendications 1 à 5, **caractérisé en ce qu'**il comprend les étapes suivantes :

- mesurer des vibrations dans la source de rayons X et dans le détecteur de rayons X ; et
- activer les premier et deuxième actionneurs afin de déplacer la source de rayons X et le détecteur de rayons X de manière à supprimer au moins une composante des vibrations.

7. Procédé selon la revendication 6, comprenant en outre l'élaboration d'un premier et d'un deuxième signal de commande pour les premier et deuxième actionneurs.

# FIG.1

# FIG.2

# FIG.3

# FIG.4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5548653 A
- US 5348124 A
- US 6959484 B
- US 20020008362 A
- US 6002778 A
- US 20020128072 A
- US 20070164189 A
- JP 2005027914 B
- JP 2003245269 B